Europäisches Patentamt

European Patent Office

Office européen des brevets

(11) Publication number: **0 180 067**
**B1**

# EUROPEAN PATENT SPECIFICATION

(45) Date of publication of patent specification: **03.01.90**

(51) Int. Cl.⁵: **A 61 L 9/01, A 61 K 7/32**

(21) Application number: **85112694.6**

(22) Date of filing: **07.10.85**

(54) Deodorant compositions containing persimmon juice as active ingredient.

(30) Priority: **08.10.84 JP 210211/84**

(43) Date of publication of application:
**07.05.86 Bulletin 86/19**

(45) Publication of the grant of the patent:
**03.01.90 Bulletin 90/01**

(84) Designated Contracting States:
**DE FR GB**

(73) Proprietor: **RILIS KAGAKU KOGYO KABUSHIKI KAISHA**
**4-7-3, Chokoji-minami**
**Toyonaka-shi Osaka-fu (JP)**

(72) Inventor: **Mimasu, Takeo**
**9, Miyano'uchi Kitsu-cho**
**Soraku-gun Kyoto-fu (JP)**
Inventor: **Torii, Kuniyoshi**
**5-10, koshien-sunada-cho**
**Nishinomiya-shi Hyogo-ken (JP)**

(74) Representative: **Vossius & Partner**
**Siebertstrasse 4 P.O. Box 86 07 67**
**D-8000 München 86 (DE)**

(56) References cited:
W.H. LEWIS et al.: "Medical Botany - Plants affecting man's health", pages 232,233,243,263, 1977, Wiley-Interscience, New York, US.
CHEMICAL ABSTRACTS, vol. 99, no. 20, 25th November 1983, page 331, abstract no. 163852n, Columbus, Ohio, US; & JP - A - 58 124 452 (SHIMADA KANKYO KAGAKU KENKYUSHO K.K.) 25-07-1983
CHEMICAL ABSTRACTS, vol. 83, no. 15, 13th October 1975, page 269, abstract no. 128751b, Columbus, Ohio, US; S.P. TANDON et al.: "Isolation and study of seed oil of Diospyros perigrina", & PROC. NATL. ACAD. SCI., INDIA, SECT. A 1974, 44, Pt. 4, 319-21

(56) References cited:
CHEMICAL ABSTRACTS, vol. 75, no. 14, 4th October 1971, abstract no. 91235t, Columbus, Ohio, US; L. OLIVEROS-BELARDO et al.: "Physical properties of some Philippine essential oils", & FLAVOUR IND. 1971, 2(5), 305-9

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

This invention relates to deodorant compositions containing, as a main component, persimmon juice. More particularly, this invention relates to the use of the pressed juice of persimmon fruits, in combination with conventional excipients, adjuvants and additives, for removing undesirable odors.

The persimmon tree is a plant of Ebenaceae which grows or is cultivated in the tropic and temperate zones of the world including Japan and China. It is said that Ebenaceae plants are classified into seven genera, and that 20 species and fruits of some kinds of persimmon trees contain, even after their maturity persimmon tannine. The persimmon tannine is known to be useful for some medicines, mordanting and leather-tannining agents.

From the EP—3—007947 it is known that dry-distillates from leaves of theaceae plants have a potent and long acting deodorizing effect. A deodorant composition containing the dry-distillates as an active ingredient is described.

The technical problem underlying the present invention is to provide a deodorant composition which is more potent in its deodorant effect than the dry-distillaits of theaceae leaves.

Thus the present invention relates to a deodorant composition containing as deodorant agent 0.01—1% by weight of the juice of the fruits of Ebenaceae plants, in combination with conventional excipients, adjuvants and additives.

In this invention, raw persimmon fruits, either sweet or astringent ones but preferably immature ones, used as a starting material material. They are pressed by an ordinary pressing machine to obtain the juice, which is converted into a powder under reduced pressure or the thus obtained pressed juice is preserved in a closed vessel for about half a year with or without enzymes, then the sugars are removed by filtration or dialysis and the residue is dried under reduced pressure to obtain a powder.

Examples

The present invention will be more fully understood with reference to the following embodiments:

Example 1

Ten kilograms of immature fruits of persimmon (Diospyros Kaki) were pressed by an ordinary pressing machine to obtain 5 kg of pressed juice, which was dried under reduced pressure, to obtain 600 g of powder.

Example 2

One kilogram of immature fruits of persimmon (Diospyros Lotus) was pressedout by an ordinary pressing machine to obtain 500 g of juice. The thus obtained juice was preserved in a closed vessel for six months to cause self-fermentation, then the sugars were removed by dialysis and the residue was dried under reduced pressure, to obtain 400 g of powder.

Examples of the deodorant effect of the active ingredient (deodorant agent) of this invention are indicated by the following experiments:

Experiment 1 — A scent test

Method;

To a solution of 0.1 g of garlic extract in 100 ml of water, each 1 ml and 2 ml of a 10% aqueous solution of the active ingredient obtained from Example 1 above were added separately. With these test solutions, six volunteers were asked to score their perception of garlic odor in the following order;

0 — Not perceptible
1 — little perceptible
2 — hardly perceptible
3 — Perceptible
4 — Strongly perceptible
5 — Very strongly perceptible

Result;

### Table 1

| No volunteer | Solution (1 ml added) | Solution (2 ml added) | Solution (Not added) |
|---|---|---|---|
| I | 2 | 0 | 5 |
| II | 3 | 1 | 5 |
| III | 2 | 0 | 4 |
| IV | 1 | 0 | 4 |
| V | 2 | 0 | 4 |
| VI | 2 | 1 | 4 |

### Experiment 2

Test solution;

Solution A; 2 w/w % aqueous solution of the active ingredient prepared according to example 1.

Solution B; 5 w/w % propylene glycol solution of the aforementioned dry-distillates obtained from theaceae leaves.

(a) Ammonia

Into two closed 500 ml vessels which contained a fixed concentration of 28% ammonia, 1 ml of Solution A and 1 ml of Solution B was added, respectively at room temperature. 10 minutes after the addition, the free ammonia gas was released from the vessel. The percentage of the ammonia which was removed by the solution added was calculated on the basis of the remaining concentration of the ammonia.

Result;

### Table 2

| Test Solu. | Concentr. at the Beginning (ppm) | Concentr. after 10 min. (ppm) | Removal Rate (%) |
|---|---|---|---|
| A | 11,000 | 3330 | 69.8 |
| B | 11,000 | 5560 | 48.7 |

(b) Trimethylamine

As in experiment (a) but using trimethylamine instead of ammonia, the following result was observed, wherein the determination of trimethylamine was carried out by gas chromatography.

### Table 3

| Test Solu. | Concentr. at the Beginning (ppm) | Concentr. after 10 min. (ppm) | Removal Rate (%) |
|---|---|---|---|
| A | 6800 | 81 | 98.8 |
| B | 6800 | 970 | 85.8 |

(c) Acetic acid

The volume of Test solutions A and B, respectively, diluted by ten times the volume required for letting disappear the odor of a fixed quantity of acetic acid was determined separately by a titration method.

It required 4 ml of Test solution A and 17 ml of Test solution B. Consequently, Test solution B only has an activity of $4/17 \times 100 = 23.5\%$ of that of Test solution A for removing the odor of acetic acid.

(d) Hydrogen sulfide

As in experiment (a) but using hydrogen sulfide instead of ammonia, the following result was observed, wherein the concentration of hydrogen sulfide was determined using an inspection tube for hydrogen sulfide.

Result;

Table 4

| Test Solu. | Concentr. at the Beginning (ppm) | Concentr. after 10 min. (ppm) | Removal Rate (%) |
|---|---|---|---|
| A | 10 | 5 | 50 |
| B | 10 | 5 | 50 |

The constituents of the active ingredients of this invention have not been definitely identified, however, they are presumed to consist of flavanols, flavonols and other higher organic molecules. Their deodorant activity may be due to a complex mechanism consisting of clathrating, addition and neutralization reactions of the active ingredient with malodorous sources in addition to biological reactions in human beings, such as inhibition of olfactory receptors.

The active ingredient of this invention can be formulated for deodorant uses in various forms with the aid of conventional excipients, adjuvants and additives, depending on location and purpose of application, some of which are illustrated as follows:

Fumigation preparations

| | |
|---|---|
| The active ingredient | 0.5 parts (w) |
| Powdered charcoal | 60 parts (w) |
| Carboxymethylcellulose | 1.0 parts (w) |
| Powdered perfume | 9.0 parts (w) |

The above components are kneaded with hot water and molded into sticks and dried.

Aerosol preparations:

| | |
|---|---|
| The active ingredient | 0.2 parts (w) |
| Dimethylether | 110 parts (w) |
| Refined water | 108 parts (w) |
| Perfume | optional |

The above components are filled into aerosol cans by a conventional method.

Granule preparations:

| | |
|---|---|
| The active ingredient | 1 part (w) |
| Lactose | 75 parts (w) |
| Dextrin | 24 parts (w) |

The above ingredients are kneaded with water, then molded by a conventional method.

Moreover, the active ingredient of this invention can be used for practical purposes in the following manner: it can be absorbed by porous unglazed plates or soaked up by papers, fibres and/or synthetic resins.

The thus produced variety of preparations are useful for deodorizing various environmental places and conditions and can be used in, for example, refrigerators, shoes, medicines and cosmetics including an underarm deodorant and anti-halitosis preparations, especially, in dealing with faeces and urine in hospitals and aged people's homes.

4

## EP 0 180 067 B1

An effective dose of the active ingredient of this invention depends, of course, upon the weight and kind of the malodorous source, however, it is tenatatively recommended to employ 0.01 to 1% by weight of the ingredient for a given weight of malodorous substances.

## Claims

1. A deodorant composition containing, as deodorant agent 0.01—1% by weight of the juice of the fruits of Ebenaceae plants, in combination with conventional excipients, adjuvants and additives.
2. The composition of claim 1, wherein said fruits are immature ones.
3. The composition of claim 1, wherein said juice is dried to form a powder.
4. A process for the preparation of a deodorant agent, according to claim 1, characterized in that the juice which is pressed out of the fruits of Ebenaceae plants is stored in a closed vessel for about six months, then the sugars are removed and the juice is dried to obtain a solid powder.

## Patentansprüche

1. Deodorant-Zusammensetzung, enthaltend, als deodoriendes Agens 0,01 bis 1 Gew.-% des Saftes der Früchte von Ebenaceae-Pflanzen in Kombination mit üblichen Träger-, Hilfs- und Zusatzstoffen.
2. Zusammensetzung nach Anspruch 1, wobei die Früchte unreif sind.
3. Zusammensetzung nach Anspruch 1, wobei der Saft zu einem Pulver getrocknet ist.
4. Verfahren zur Herstellung eines deodorierenden Agens nach Anspruch 1, dadurch gekennzeichnet, daß der Saft, der aus den Früchten von Ebenaceae-Pflanzen gepreßt wird, in einem geschlossenen Gefäß etwa 6 Monate lang gelagert, dann die Zucker entfernt und der Saft zu einem festen Pulver getrocknet wird.

## Revendications

1. Composition déodorante contenant, comme agent déodorant, 0,01—1% en poids du jus de fruits de plantes ébénacées, en combinaison avec des excipients, des adjuvants et des additifs classiques.
2. Composition selon la revendication 1, dans laquelle lesdits fruits ne sont pas mûrs.
3. Composition selon la revendication 1, dans laquelle ledit jus est séché our donner une poudre.
4. Procédé pour la préparation d'un agent déodorant selon la revendication 1, caractérisé en ce que le jus obtenu pressage des fruits de plantes ébénacées, est stocké dans un récipient fermé pendant environ six mois, puis en ce que les sucres sont éliminés et en ce que le jus est séché our donner une poudre solide.